Europäisches Patentamt

European Patent Office

Office européen des brevets

(19)

(11) Publication number: **0 074 411**

**B1**

(12) **EUROPEAN PATENT SPECIFICATION**

(45) Date of publication of patent specification: **28.05.86**

(21) Application number: **82900989.3**

(22) Date of filing: **19.03.82**

(86) International application number:
**PCT/JP82/00076**

(87) International publication number:
**WO 82/03393 14.10.82 Gazette 82/25**

(51) Int. Cl.⁴: **C 07 D 307/62, A 61 K 31/34**

(54) ASCORBIC ACID DERIVATIVES.

(30) Priority: **24.03.81 JP 43044/81**

(43) Date of publication of application:
**23.03.83 Bulletin 83/12**

(45) Publication of the grant of the patent:
**28.05.86 Bulletin 86/22**

(84) Designated Contracting States:
**FR**

(56) References cited:
**JP-A-49 087 655**
**US-A-4 208 434**
**US-A-4 208 434**

**CHEMICAL ABSTRACTS, vol. 55, no. 16, August 7, 1961, column 15698e COLUMBUS OHIO (US) A.B. KATSHEL'SON et al.: "Vitamin C in normal and diseased human cornea"**

**Journal of Agricultural & Food Chemistry, Vol. 28, No. 6, November/December. 1980 (Columbus, Ohio) Keki R. Bharucha et al.**

**"Long-Chain Acetals of Ascorbic and Erythorbic Acids as Antinitrosamine Agents for Bacon" p. 1274 - 1281, Especially, P. 1274, left column**

(73) Proprietor: **SANTEN PHARMACEUTICAL CO., LTD.**
**9-19, 3-Chome, Shimoshinjo**
**Higashiyodogawa-ku Osaka533 (JP)**

(72) Inventor: **IWAO, Jun-ichi**
**7-27, Nogami 4-chome Takarazuka-shi**
**Hyogo 665 (JP)**
Inventor: **OYA, Masayuki**
**27-18, Yamatedai 3-chome**
**Ibaraki-shi Osaka 567 (JP)**
Inventor: **ISO, Tadashi**
**197-7, Joroku Sakai-shi**
**Osaka 588 (JP)**

(74) Representative: **Bloch, Robert et al**
**Cabinet ROBERT BLOCH 39 avenue de Friedland**
**F-75008 Paris (FR)**

(56) References cited:

**Yakugaku Zasshi, Vol. 86, No. 5, May. 1966 (Tokyo), Tanaka Yoshihiro hoka "Ascorbic Acid Yudotai no Seizaigakuteki Kenkyu (Dai Ippo)" P. 376-383**

**CHEMICAL ABSTRACTS vol.55 (1961), 15698e**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

Technical field of the invention
This invention relates to 3,6—O,O' - dipivaloyl - L - ascorbic acid and salts thereof.

Background of the invention
Ascorbic acid is contained in the eye lens and aqueous humor in higher concentration than in other tissues (the usual level in the lens is 20 to 30 mg %).
The lenticular ascorbic acid as well as glutathion is considered to contribute to the oxidation-reduction system in the lens and functionally to maintain the lens transparency. The resolution of the ascorbic acid concentration in cataractous lens have been observed by many investigators.
From the above fact, ascorbic acid introduced to the eyes or given orally is thought to be useful to prevent the cataracts formation. But the solubility of ascorbic acid in lipoids is very small, so its practical use for therapeutics is limited.
Although a number of esters of ascorbic acid, for example acetylascorbic acid, benzoylascorbic acid, palmitoylascorbic acid, are known, the utility of these compounds for medical drugs is limited because of their low solubility in lipoids and low membrane permeability.

Description of the invention
We succeeded to improve the stability, solubility in lipoids and membrane permeability of ascorbic acid by the conversion to a particular pivalate, and thus we found that the 3,6—O,O' - dipivaloyl - L - ascorbic acid and its salts were useful as therapeutic agent for cataracts.
Many acyl derivatives have been described in particular in Yakugaku Zasshi *86* No. 5 (1966) page 376—383 and JP—A—4 987 655 and even mono-pivaloyl derivates for instance in J. Agricultural & Food Chemistry *28* (1980) page 1274—1281 and US—A—4 208 434 but none of said references describes the di-pivaloyl derivate of the invention which shows a particularly good effect.
The compound of this invention can be prepared, for example, by
A) the reaction of L-ascorbic acid with active derivatives of pivalic acid, for example pivaloyl chloride; and
B) the rearrangement of 2,6—O,O' - dipivaloyl - L - ascorbic acid to 3,6—O,O' - dipivaloyl - L - ascorbic acid in the presence of pyridine.
Solubility test, acute toxicity test and examples of formulation are shown below.

Solubility test
As a lipophilic scale, the partition coefficient in a water-octanol system was measured.

Experimental method
To 3—4 mg of the compound of this invention or the control compound (L-ascorbic acid), 10 ml of water and octanol are added respectively and well shaked. The resulting mixture is centrifuged and the contents of the compound of this invention and control compound in water or octanol layer were measured by high performance liquid chromatography.

Result

|  | Peak height | |
| --- | --- | --- |
|  | octanol layer | water layer |
| 3,6—O,O'-dipivaloyl-L-ascorbic acid | 54.8 | 69.8 |
| 2,6—O,O'-dipivaloyl-L-ascorbic acid | 143.4 | 0.2 |

Acute toxicity test
Acute toxicity of 3,6—O,O' - dipivaloyl - L - ascorbic acid of this invention is shown below.
Animal: ddY-SPF strain male mouse (weight: 25 to 30 g)
Method of administration: The compound is administered orally as 0.5% tragacanth suspension.
Acute toxicity: $LD_{50}$ 5000 mg/kg.
The compound of this invention can be administered either orally or parenterally. The dosage forms are tablet, capsule, eye drops, etc.
The dosage is adjusted depending on symptoms or dosage forms, and in oral case usual daily dosage is 1 to 5000 mg, preferably 10 to 1000 mg, in one or few divided doses.
In case of eye drops, 2 to 3 drops are introduced to the eyes daily several times.

Examples of the formulation:

(1) Oral drug

Tablet

| | |
|---|---|
| — 3,6—O,O'-Dipivaloyl-L-ascorbic acid | 100 mg |
| — ethyl cellulose | 50 mg |
| — crystalline cellulose | 80 mg |
| — carboxymethyl cellulose | 7 mg |
| — magnesium stearate | 3 mg |
| Total | 240 mg |

(2) Eye drops

The content of 3,6—O,O' - dipivaloyl - L - ascorbic acid is 5 mg in 5 ml of the aqueous solution of 3,6—O,O' - dipivaloyl - L - ascorbic acid.

Example 1

3,6—O,O'-Dipivaloyl-L-ascorbic acid

3.5 g of L-ascorbic acid is added to 30 ml of N,N - dimethylformamide, and 4.8 ml of pyridine is added to form a solution. 4.8 g of pivaloyl chloride is added dropwise to the solution with stirring. After the addition the solution is stirred for 3 hours at room temperature. The reaction mixture is concentrated in vacuo and to the resulting oily residue water is added and stirred.

The mixture is allowed to stand, and then the water layer is removed by decantation. The residue is purified by silica gel column chromatography to give 1.2 g (18%) of the title compound.

mp 149.5—152°C (chloroform n-hexane)

$(\alpha)_D^{24}$+43.8° (c=1.0, methanol)

IR 3465, 1764, 1740, 1735, 1701, 1640, 1289, 1146, 1128

NMR (CDCl$_3$, δ)

1.21 (9H, s, —OCOC(CH$_3$)$_3$), 1.30 (9H, s, —OCOC(CH$_3$)$_3$), 4.27 (3H, s, C$_5$—H and C$_6$—H$_2$), 4.83 (1H, s, C$_4$—H), 5.31—7.08 (2H, Br, —OH×2)

Example 2

3,6—O,O'-Dipivaloyl-L-ascorbic acid

45 g of 2,6—O,O' - dipivaloyl - L - ascorbic acid is dissolved in 400 ml of dioxane and 53 ml of pyridine is added.

The reaction mixture is stirred for 1.5 hours at room temperature and concentrated in vacuo. To the resulting residue dilute hydrochloric acid and ethyl acetate are added, and the organic layer is washed with saturated sodium chloride solution and dried over anhydrous magnesium sulfate. The solvent is removed off in vacuo to give 31.8 g (71%) of the title compound.

The physical constants of the obtained crystals are the same as those of Example 1.

Example of preparation of the 2,6—O,O'-dipivaloyl-L-ascorbic acid,

which is not claimed, but serves as a starting product to produce the 3,6-derivate according to the above example 2.

5.0 g of 5,6—O - isopropylidene - L - ascorbic acid is added to 200 ml of acetone, and 4.7 ml of pyridine is added to form a solution. 13.9 g of pivaloyl chloride is added dropwise to the solution with stirring. After the addition, the solution is stirred for one hour at room temperature, and then 20 ml of methanol is added and further stirred for ten minutes. The reaction mixture is concentrated in vacuo, and to the resulting oily residue water is added to produce crystals, which are collected by filtration to give 5.4 g (68%) of the 2,6—O,O' - dipivalyl - L - ascorbic acid.

**Claims**

1. 3,6—O,O' - Dipivaloyl - L - ascorbic acid and salts thereof.

2. A process for preparing the compound of claim 1, by the reaction of L-ascorbic acid with active derivatives of pivalic acid, for example pivaloyl chloride.

3. A process for preparing the compound of claim 1, by the rearrangement of 2,6—O,O' - dipivaloyl - L - ascorbic acid, in the presence of pyridine.

4. Therapeutic agent for cataracts comprising a compound as in claim 1.

## 0 074 411

**Patentansprüche**

1. 3,6—O,O' - Dipivaloyl - L - Ascorbinsäure und deren Salze.

2. Verfahren zur Herstellung der Verbindung nach Anspruch 1 durch Umsetzung von L-Ascorbinsäure mit aktiven Derivaten der Pivalinsäure, beispielsweise Pivaloylchlorid.

3. Verfahren zur Herstellung der Verbindung nach Anspruch 1 durch Umgruppierung von 2,6—O,O' - Dipivaloyl - L - Ascorbinsäure in Gegenwart von Pyridin.

4. Therapeutisches Mittel zur Kataraktbehandlung, enthaltend eine Verbindung nach Anspruch 1.

**Revendications**

1. Acide 3,6—O,O' - dipivaloyl - L - ascorbique et ses sels.

2. Procédé pour la préparation du composé de la revendication 1, par réaction d'acide L-ascorbique avec des dérivés actifs d'acide pivalique, par exemple chlorure de pivaloyle.

3. Procédé pour la préparation du composé de la revendication 1, par réarrangement d'acide 2,6—O,O' - dipivaloyl - L - ascorbique en présence de pyridine.

4. Agent thérapeutique pour les cataractes comprenant un composé selon la revendication 1.